# EUROPÄISCHE PATENTANMELDUNG

(11) **EP 2 108 446 A1**
(43) Veröffentlichungstag der Anmeldung: **14.10.2009**
(21) Anmeldenummer: 08006984.2
(22) Anmeldetag: 08.04.2008
(51) Int. Cl.: B01J 20/22, A61L 9/014, A61L 9/01, B01D 53/02, C11D 3/00

(54) **Feste Geruchsadsorber auf der Basis von Zinkricinoleaten und verwandten Verbindungen**

(71) Anmelder: SÜD-CHEMIE AG, 80333 München (DE)
(72) Erfinder: Sohling, Ulrich, 85356 Freising (DE); Ruf, Friedrich, 84184 Ast (DE); Kuhn, Herbert, 42697 Sohlingen (DE); Thie, Gordon, 45527 Hattingen (DE)
(74) Vertreter: Stolmár, Matthias

(57) **Zusammenfassung**

Die Erfindung betrifft Zusammensetzungen zur Adsorption von Schadstoffen und/oder Geruchsstoffen, wobei die Zusammensetzung im wesentlichen wasserfrei ist und die folgenden Komponenten enthält: a1) mindestens ein Metallion in Form eines Ligandenkomplexes, und a2) mindestens einen Lösungsvermittler, in dem der Ligandenkomplex des Metallions vollständig löslich ist; oder b) ein Zinkethercarboxylat. Weiterhin sind Verfahren zur Herstellung sowie die Verwendung solcher Zusammensetzungen offenbart.

## Beschreibung

Gegenstand der vorliegenden Erfindung sind Zusammensetzungen mit Schadstoffe bzw. Geruchsstoffe-adsorbierender Wirkung, enthaltend Zinkricinoleat oder verwandte Verbindungen, wobei die Zusammensetzungen im Wesentlichen wasserfrei sind.

In vielen Bereichen der Technik stellt sich das Problem einer effizienten Geruchsstoff- oder Schadstoffbindung. So können bspw. Gerüche aus unterschiedlichsten Quellen stammen. Viele Gerüche natürlichen Ursprungs stammen von Mensch oder Tier oder aus landwirtschaftlichen oder industriellen Produktionsprozessen und können unmittelbar oder über die entsprechenden Produkte an die Umgebung abgegeben werden.

Im Stand der Technik sind zahlreiche Mittel und Verfahren zur Unterdrückung oder Entfernung von Gerüchen, insbesondere von unangenehm empfundenen Gerüchen beschrieben. Ein Teil der bekannten Verfahren beschränkt sich auf die Maskierung solcher Gerüche, d.h. die Überdeckung durch als angenehm empfundene Duftstoffe. Ein anderer Teil des Standes der Technik beschäftigt sich mit der irreversiblen Bindung von Geruchsstoffen durch chemische Reaktionen oder physikalische Adsorption und Absorption an geeigneten Materialien. Aus verständlichen Gründen ist die irreversible Bindung der Geruchsstoffe der effektivere und länger anhaltende Weg. Aus dem Stand der Technik sind eine Reihe von als Geruchsabsorber verwendeten Aktivstoffen, wie z. B. Aktivkohle bekannt.

Die EP 1 319 394 A1 beschreibt Zubereitungen mit desodorierender Wirkung, enthaltend (a) das Zinksalz der Ricinolsäure, (b) aminofunktionelle Aminosäuren, (c) Lösungsvermittler, (d) organische und/oder anorganische Säuren und (e) Wasser. Dabei wurde gefunden, dass durch die Mitverwendung von aminofunktionellen Aminosäuren, deren Salze und/oder Derivaten Mittel mit desodorierender Wirkung erhalten werden, welche verbesserte Eigenschaften aufweisen.

Es besteht ein starker Bedarf nach weiter verbesserten Geruchsstoffe und/oder Schadstoffe adsorbierenden Zusammensetzungen, die eine möglichst effiziente und lang anhaltende Wirkung aufweisen.

Es ist daher eine Aufgabe der vorliegenden Erfindung, solche Zusammensetzungen mit möglichst vorteilhafter desodorierender Wirksamkeit zur Verfügung zu stellen.

Dabei wurde im Rahmen der vorliegenden Erfindung überraschend gefunden, dass sich besonders wirksame Schadstoffe und/oder Geruchsstoffe adsorbierende Zusammensetzungen erhalten lassen, wenn darin neben Zinkricinoleat oder verwandten Metallsalz-Liganden-Verbindungen mindestens ein nicht wässriger Lösungsvermittler enthalten ist, indem der Metallion-Ligandenkomplex löslich ist, wobei die Zusammensetzung im Wesentlichen wasserfrei ist. Im folgenden wird der Ausdruck Zinkricinoleat stellvertretend auch für die anderen in der vorliegenden Beschreibung offenbarten Metallion-Ligandenkomplexe verwendet.

Ein erster Aspekt der vorliegenden Erfindung betrifft somit eine Zusammensetzung zur Adsorption von Schadstoffen und/oder Geruchsstoffen, wobei die Zusammensetzung im wesentlichen wasserfrei ist und die folgenden Komponenten enthält:
a1) mindestens ein Metallion in Form eines Ligandenkomplexes, und
a2) mindestens einen Lösungsvermittler, in dem der Ligandenkomplex des Metallions vollständig löslich ist.

Ein weiterer Aspekt der vorliegenden Erfindung betrifft eine Zusammensetzung zur Adsorption von Schadstoffen und/oder Geruchsstoffen, wobei die Zusammensetzung im wesentlichen wasserfrei ist und mindestens ein Zinkethercarboxylat enthält. Das Zinkethercarboxylat kann vorzugsweise alternativ zu den Komponenten a1) und a2) vorhanden sein, oder nach einer möglichen Ausführungsform auch zusätzlich.

Es wurde im Rahmen der vorliegenden Erfindung unerwartet gefunden, dass durch die Anwesenheit von Wasser bereits ab einem geringen Anteil der Gesamtzusammensetzung die Bereitstellung des Zinkricinoleats erheblich gestört wird.

Ohne dass die Erfindung auf die Richtigkeit dieser Annahme beschränkt wäre, wird als theoretischer Wirkmechanismus angenommen, dass durch die erfindungsgemäß verwendeten nicht-wässrigen Lösungsvermittler eine effiziente Adduktbildung zwischen dem Lösungsvermittler und dem Zinkricinoleat erfolgt, wobei das Zinkion für eine effiziente Wirkung zur Schadstoff- bzw. Geruchsstoffneutralisierung optimal positioniert bzw. "aktiviert" ist. Diese Adduktbildung kann bereits durch die Anwesenheit von einem geringen Anteil an Wasser empfindlich gestört werden. Dabei kann, ohne dass die Erfindung auf diesen theoretischen Mechanismus beschränkt wäre, die Ausbildung von Wasserstoffbrückenbindungen durch die störenden Wassermoleküle im unmittelbaren Umfeld um das Zinkion angenommen werden, wobei eine vorteilhafte nahe Anlagerung der Lösungsvermittlermoleküle an das Zink beeinträchtigt bzw. verhindert wird. Auch im Falle des Zinkethercarboxylats beeinträchtigt die Anwesenheit von bereits geringen Mengen an Wasser bereits die Wirksamkeit der Komponenten, oder verhindert diese sogar vollständig.

Erfindungsgemäß liegt der Anteil an Wasser bezogen auf die Gesamtzusammensetzung bei weniger als 5 Gew.-%, insbesondere weniger als 4 Gew.-%, insbesondere weniger als 3 Gew.-%, insbesondere weniger als 2 Gew.-%, insbesondere weniger als 1 Gew.-%, insbesondere weniger als 0,5 Gew.-%, insbesondere weniger als 0,2 Gew.-%, insbesondere weniger als 0,1 Gew.-%.

Nach einer bevorzugten erfindungsgemäßen Ausführungsform enthält die erfindungsgemäße Zusammensetzung eine Verbindung der nachstehenden Formel (I): ${L}_{y} - Zn - {\left(HOR\right)}_{x}$
wobei L für einen Liganden für das Zinkion und R-OH für einen Alkohol stehen. x und y stellen positive Zahlen dar.

Alternativ zu Zink können, wie bereits vorstehend erwähnt, auch Ionen anderer Metalle eingesetzt werden, für die im Stand der Technik die Eignung zur Geruchsstoff- bzw. Schadstoffbindung beschrieben wurde, wie z. B. Kupfer, Eisen, Cadmium, Quecksilber, Molybdän, Blei, Cobalt, Nickel, Chrom, Vanadium und Wolfram.

Erfindungsgemäß können als Liganden allgemein solche organischen Verbindungen verwendet werden, die mit dem Zink (bzw. dem anderen Metallion) ligieren und in dem Lösungsvermittler in Form des Metallion-Ligandenkomplexes vollständig löslich sind.

Beispiele solcher Liganden sind hydroxylierte, ungesättigte und/oder gesättigte Fettsäuren wie insbesondere Ricinoleat, oder Acetat und dergleichen. Liganden sind allgemein funktionalisierte Fettsäuren, die folgende funktionelle Gruppen enthalten können: -OH, -SO₃⁻, SO₃H, -SO₄⁻, SO₄H, -COO⁻, -COOH, -PO₃⁻ PO₃H, PO₄⁻, PO₄H, -O-(C-C-O-)ₙ-C-C-OH (n=1, 2, ....N), -SH, -S⁻, -SR, NO₂, NO₃⁻, -NH₂, -NHR, -NR₂, -NH₃⁺, -NH₂R⁺, -NHR₂⁺, Glykole, Glycerin, Polyglykole, Polyglycerine, F, Cl, I, Br. Liganden sind auch Sulfate, z.B. Ethersulfate, Sulfonate, Phosphate und Phosphonate.

Erfindungsgemäß können die Zusammensetzungen zur Neutralisierung bzw. Adsorption beliebiger Schadstoffe oder Geruchsstoffe verwendet werden, wobei beispielhaft und ohne Beschränkung Ammoniak, Schwefelwasserstoff, Mercaptane wie Ethylmercaptan, Amine, Thiole, Thioether, Carbonsäuren, NOₓ oder auch ionische Verbindungen wie NO₃⁻, NO₂⁻, PO₄³⁻, H₂PO₄⁻, HPO₄²⁻ genannt werden können. Weitere mögliche Schadstoffe sind Sulfonate, Sulfite und Sulfate, aliphatische und aromatische Phosphorverbindungen.

Der Begriff Adsorption soll dabei auch eine Absorption, Chemisorption oder anderweitige Bindung umfassen.

Geeignete Lösungsvermittler sind, ohne das die nachstehende Auflistung beschränkend wäre, Alkohole, nichtionische und ionische Tenside wie Alkoxilate, Polyglycerine, Glykolether, Glykole, Polyethylenglykole, Polypropylenglykole, Polybutylenglykole, Glycerinesterthoxylate, Polysorbate, Alkylethersulfaten, Alkyl- und/oder Arylsulfonaten, Akylsulfaten, Estersulfonate (Sulfofettsäureester), Lingninsulfonate, Fettsäurecyanamide, anionische Sulfobernsteinsäuretenside, Fettsäureisethionate, Acylaminoalkansulfonate (Fettsäuretauride), Fettsäuresarcosinate, Ethercarbonsäuren und Alkyl(ether)-Phosphate. Bevorzugte nichtionische Lösungsvermittler sind die, gegebenenfalls einseitig mit einem C₁-C₆-Alkanol veretherten, C₂-C₆-Alkylenglykole und Poly-C₂-C₃-alkylenglykolether mit durchschnittlich 1 bis 9 gleichen oder verschiedenen, vorzugsweise gleichen Alkylenglykolgruppen pro Molekül wie auch Alkohole und Fettalkoholpolyglykolether, vorzugsweise Propylenglykol, Dipropylenglykol, Trimethylolpropan sowie niederethoxyilierte Fettalkohole mit 6 bis 22, vorzugsweise 8 bis 18, insbesondere 8 bis 12 und besondere bevorzugt 8 bis 11 Kohlenstoffatomen.

Nach einer besonders bevorzugten Ausführungsform wurde gefunden, dass als Lösungsvermittler primäre oder sekundäre Alkohole mit 1 bis 4 Kohlenstoffatomen, insbesondere um Ethanol oder Methanol besonders vorteilhaft sind.

Die Zusammensetzungen können entweder direkt in im wesentlichen wasserfreier Form eingesetzt werden, oder es können beliebige Festkörper als Träger verwendet und insbesondere mit den erfindungsgemäßen Zusammensetzungen beschichtet werden. Beispiele für geeignete feste Trägermaterialien sind, ohne Beschränkung, Glas (Poraver), natürliche und synthetische Schichtsilicate, Kieselgele, Aluminiumoxide, Titanoxide, Aktivkohle, poröse Kunststoffe und dergleichen, sowie Mischungen davon. Unter den Schichtsilicaten können beispielsweise dass solche aus der Gruppe der Zweischichtsilicate, insbesondere Chyrsotil, Antigorit, Kaolinit, Halloysit und Dreischichtsilicate, insbesondere Talk, Pyrophyllit, Saponit, Hectorit, Montmorillonit, Beidellit, Nontronit, Biotit, Phlogopit, Lepidolith, Muskovit, Paragonit, Margarit und Chlorit, oder deren Mischungen ausgewählt werden.

Gemäß der vorliegenden Erfindung umfasst der Ausdruck "Schichtsilicat" auch schichtsilicathaltige Zusammensetzungen, d.h. Zusammensetzungen, die neben einem Schichtsilicatanteil auch einen oder mehrere andere Anteile bzw. Komponenten aufweisen. Nach einer bevorzugten Ausführungsform handelt es sich bei den schichtsilicathaltigen Zusammensetzungen um solche, die eine Mischung mit einem Schichtsilicatanteil und einer amorphen Phase aufweisen, insbesondere eine Schichtsilicat-Kieselgel-Mischphase.

Nach einer vorteilhaften erfindungsgemäßen Ausführungsform weist die schichtsilicathaltige Zusammensetzung eine BET-Oberfläche von mehr als 120 m²/g, ein Gesamtporenvolumen von mehr als 0,35 ml/g und einen Siliciumgehalt, berechnet als SiO₂, von mindestens 60 Gew.% auf. Vorzugsweise sind die Plättchen bzw. Plättchenstapel des Schichtsilicats im Wesentlichen homogen in einer kontinuierlichen amorphen Silica-Phase verteilt bzw. festgelegt. Es kann sich erfindungsgemäß um ein natürliches oder synthetisches Material handeln. Nach einer bevorzugten Ausführungsform enthält die schichtsilicathaltige Zusammensetzung mindestens 1 Gew.-%, insbesondere mindestens 5 Gew.-%, weiter bevorzugt mindestens 10 Gew.-%, einer amorphen Phase. Die amorphe Phase wird vorzugsweise von SiO₂ gebildet. Vorzugsweise besteht der Schichtsilicatanteil der schichtsilicathaltigen Zusammensetzung aus einem Smektit. Die schichtsilicathaltige Zusammensetzung enthält nach einer möglichen Ausführungsform weniger als 70 Gew.-%, weiter bevorzugt weniger als 60 Gew.-% einer Smektitphase. Die schichtsilicathaltige Zusammensetzung enthält nach einer weiteren möglichen Ausführungsform mindestens 10 Gew.-%, weiter bevorzugt mindestens 20 Gew.-% einer Smektitphase. Das Verhältnis von Smektitphase zu amorpher Phase liegt nach einer bevorzugten Ausführungsform im Bereich zwischen 2 und 0,5, weiter bevorzugt zwischen 1,2 und 0,8. Neben der amorphen Phase und der Smektitphase können weitere Mineralphasen in der Zusammensetzung enthalten sein, vorzugsweise im Bereich von 0,5 bis 40 Gew.%, insbesondere bevorzugt zwischen 1 und 30 Gew.%. Beispiele solcher Mineralphasen sind Quarz, Kristoballit, Feldspat und Calcit. Andere Nebenmineralien können auch vorhanden sein. Nach einer weiteren bevorzugten Ausführungsform sind die eingesetzten schichtsilicathaltigen Zusammensetzungen kaum quellbar. Vorzugsweise haben diese Zusammensetzungen ein Sedimentvolumen in Wasser nach einer Stunde von weniger als 15 ml/2 g, weiter bevorzugt von weniger als 10 ml/2 g. Weiterhin wird bevorzugt, dass die Zusammensetzung eine Kationenaustauschkapazität von mehr als 40 meq/100 g, weiter bevorzugt mehr als 45 meq/100 g aufweisen. Nach einer besonders bevorzugten Ausführungsform wird als schichtsilicathaltige Zusammensetzung ein Material wie in der internationalen Patentanmeldung PCT/EP2007/009656 A der gleichen Anmelderin beschrieben eingesetzt. Die diesbezügliche Offenbarung wird ausdrücklich durch in Bezugnahme in die vorliegende Beschreibung aufgenommen.

Nach einer vorteilhaften Ausführungsform weist das verwendete Schichtsilicat folgende BET Oberflächen auf: > 10 m²/g, bevorzugt > 20 m²/g , besonders bevorzugt > 50 m²/g. Weiter wird bevorzugt, dass der mittlere Porendurchmesser, bestimmt nach der BJH-Methode über den Bereich von 1,7 bis 300nm, bei mehr als 8 nm, insbesondere mehr als 9 nm liegt.

Die erfindungsgemäßen Zusammensetzungen können, gegebenenfalls nach der Aufbringung auf einen festen Träger sowohl zur Neutralisierung bzw. Adsorption von Schadstoffen und/oder Geruchsstoffen aus gasförmigen Medien wie Luft oder aus flüssigen Medien wie wässrigen Systemen eingesetzt werden.

Nach einer besonders bevorzugten Ausführungsform werden die erfindungsgemäßen Zusammensetzungen, insbesondere in geträgerter Form, zur Adsorption von Ammoniak und/oder Schwefelwasserstoff, oder auch Nitrat aus wässrigen Medien eingesetzt.

Die erfindungsgemäßen Zusammensetzungen gemäß der Variante a), d.h. mit den Komponenten a1) und a2) wie hierin beschreiben können beispielsweise hergestellt werden, indem eine alkoholische Lösung der entsprechenden Zink-Ligandenkomponente (ZnLₓ) in Alkohol unter weitgehendem Ausschluss von Wasser (siehe oben), z. B. von Ethanol mit einem Reinheitsgrad von größer 99,8 %, hergestellt wird. Somit betrifft ein weiterer Aspekt der vorliegenden Erfindung ein Verfahren zur Herstellung einer Zusammensetzung zur Adsorption von Schadstoffen und/oder Geruchsstoffen, umfassend mindestens die folgenden Schritte:
a) Bereitstellen mindestens eines Metallions in Form eines Ligandenkomplexes, und
b) Bereitstellen mindestens eines Lösungsvermittlers, in dem der Ligandenkomplex des Metallions vollständig löslich ist;
c) In Kontaktbringen der Komponenten a) und b).

Nicht beschränkende Beispiele besonders geeigneter Verbindungen der vorstehenden Formel I sind Addukte aus Zinkacetat mit Methanol, Ethanol, einen C3-Alkohol oder einem C4-Alkohol, Addukte aus CuCl₂ und eine C1- bis C4-Alkohol, sowie Addukte aus Zinkricinoleat und einem C1- bis C12-Alkohol.

Nach einer weiteren erfindungsgemäßen Ausführungsform enthält die erfindungsgemäße Zusammensetzung anstelle oder zusätzlich zu der Verbindung gemäß Formel I eine Verbindung der folgenden Formel II
[R-(O-C-C-)ₙ-COO]₂Zn,
wobei R einen aliphatischen oder cyclischen bzw. aromatischen organischen Rest darstellt, insbesondere eine aliphatische gesättigte oder ungesättigte C-Kette oder einen aromatischen organischen Rest. n stellt eine positive Zahl (1, 2, ...) dar. (O-C-C-)ₙ stellt ein Polyethylenoxid dar.

So wurde nach einem weiteren Aspekt der vorliegenden Erfindung überraschend gefunden, dass solche Zinkethercarboxylate bei weitgehender Abwesenheit von Wasser hervorragende Wirkstoffe zur Geruchsstoff- und/oder Schadstoffbildung darstellen.

Diese Verbindungen lassen sich beispielsweise herstellen, indem basisches Zinkcarbonat (ZnCO₃/Zn(OH)₂) in einer Ethercarbonsäure gelöst wird. Dabei kann folgende Reaktionsgleichung angenommen werden: 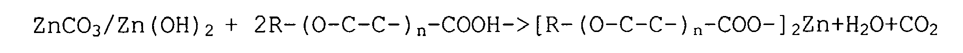 Es entsteht eine klare Lösung (pH=4-5), die mit Wasser beliebig verdünnbar ist. Es wurde jedoch überraschend gefunden, dass nur das im wesentlichen wasserfreie Konzentrat aktiv gegenüber Schadstoffen bzw. Geruchsstoffen ist. Somit betrifft ein weiterer Aspekt der vorliegenden Erfindung ein Verfahren zur Herstellung einer Zusammensetzung zur Adsorption von Schadstoffen und/oder Geruchsstoffen, umfassend mindestens die folgenden Schritte:
a) Bereitstellen eines basischen Zinkcarbonats, und
b) Lösen der Komponente a) in mindestens einer Ethercarbonsäure.

Ein nicht beschränkendes Beispiel einer besonders bevorzugten Ethercarbonsäure ist

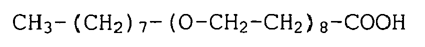

Die vorstehenden Verbindungen der Formel II können grundsätzlich zur Neutralisierung bzw. Adsorption der gleichen Schad- und Geruchsstoffe verwendet werden, wie die vorstehenden Verbindungen der Formel I.

Dabei werden die Verbindungen nach einer bevorzugten erfindungsgemäßen Ausführungsform als wasserfreie Lösung bzw. Konzentrat auf einen beliebigen festen Träger (siehe oben) aufgebracht. Nach einer anderen bevorzugten erfindungsgemäßen Ausführungsform kann die Verbindung jedoch auch in trockenem wasserfreiem Zustand (ohne Träger) oder in Form der im wesentlichen wasserfreien Lösung bzw. des im wesentlichen wasserfreien Konzentrats eingesetzt werden.

Nach einem alternativen erfindungsgemäßen Aspekt der Erfindung lässt sich die Herstellung des Aktivstoffes (d.h. der Zusammensetzung ohne den Träger (z.B. Schichtsilicat)) und die sich daran anschließende Belegung des Trägers direkt aus den Rohstoffen:
Ligand (z.B. Fettsäure), Metallsalz (z.B. ZnSO₄) und der alkoholischen Adduktkomponente (Lösungsvermittler, z.B hochsiedender
Fettalkohol), "in-situ" durchführen. Dabei erfolgt die Herstellung des Aktivstoffes, insbesondere des Komplexes mit dem Metallion und dem Liganden, zunächst in wässriger Lösung. Weiterhin wird ein Lösungsvermittler (Adduktbildner) wie hierin beschrieben zugegeben. Anschließend wird, soweit zu Herstellung einer festen Zusammensetzung wie eines Pulvers erwünscht, ein Träger zugegeben. Vorzugsweise setzt man hier Alkohole als Adduktkomponenten ein, die einen höheren Siedepunkt als Wasser aufweisen (hier als "höhersiedend" bezeichnet). Nach dem Verdampfen des
Wassers erhält man dann (auf dem Trägermaterial bzw. in Abwesenheit eines Trägers als Lösung) das wasserfreie Wirkstoff-Addukt. Dieses erfindungsgemäße Material ist ebenso aktiv bezüglich der
Schadstoff- und/oder Geruchsstoffsorption wie die nach dem vorher angeführten Verfahren hergestellten erfindungsgemäßen Materialien.

Somit betrifft ein weiterer erfindungsgemäßer Aspekt ein Verfahren, worin die Bereitstellung des Komplexes mit dem Metallion und dem Liganden, (zunächst) in wässriger bzw. wasserhaltiger Lösung erfolgt. Vorzugsweise wird hierzu ein Salz des Metallions in wässriger Lösung mit dem Liganden umgesetzt. Weiterhin, insbesondere anschließend, wird mindestens ein Lösungsvermittler (Adduktbildner) zugegeben. Das Wasser wird anschließend weitgehend (entsprechend der hierin beschriebenen Grenzen für den Wasseranteil der Gesamtzusammensetzung) oder vollständig entfernt, insbesondere abgedampft, wobei sich vorzugsweise das Addukt des Lösungsvermittlers mit dem Metallion-Ligandenkomplex bildet. Das Entfernen von Wasser erfolgt vorzugsweise, jedoch nicht zwingend (z.B. bei der Herstellung einer flüssigen erfindungsgemäßen Zusammensetzung) in Anwesenheit mindestens eines Trägers wie hierin beschrieben.

Vorzugsweise wird ein Lösungsvermittler eingesetzt, der einen höheren Siedepunkt aufweist als Wasser, insbesondere ein hochsiedender Alkohol, vorzugsweise ein Fettalkohol.

Nach einer erfindungsgemäßen Ausführungsform wird die erfindungsgemäße Zusammensetzung nach der Herstellung getrocknet und gegebenenfalls zu Granulat, Presslingen oder Tabletten geformt.

Es wurde im Rahmen der vorliegenden Erfindung auch überraschend gefunden, dass in den erfindungsgemäßen Zusammensetzungen die Anwesenheit von aminofunktionellen Amminosäuren nicht erforderlich oder sogar schädlich, und einer effizienten Wirksamkeit zur Geruchsstoff- und/oder Schadstoffadsorption abträglich ist.

Somit besteht nach einer besonders bevorzugten erfindungsgemäßen Ausführungsform die erfindungsgemäße Zusammensetzung zu mehr als 90 Gew.-%, insbesondere mehr als 95 Gew.-%, weiter bevorzugt mehr als 98 Gew.-%, noch weiter bevorzugt mehr als 99 Gew.-% und weiter bevorzugt mehr als 99,5 Gew.-% aus den Komponenten a1) und a2) bzw. b) wie hierin beschreiben, bei einem Gesamtwassergehalt, der die vorstehend angegebenen Werte nicht überschreitet, und ggf. einen Träger.

Ein weiterer erfindungsgemäßer Gegenstand betrifft die Verwendung der vorstehenden Zusammensetzung zur Herstellung von desodorierend oder Schadstoffe neutralisierenden bzw. adsorbierenden hygienischen und/oder kosmetischen Formulierungen, Haushaltsreinigern, Industriereinigern, Adsorbern in Filtern, Formulierungen für die Anwendung in der privaten und gewerblichen Tierhaltung, Formulierungen für die Behandlung von textilen Fasern und Geweben, Waschmittelzusammensetzungen und dergleichen.

Die Zinksalze der Ricinolsäure bzw. die entsprechenden Metallion-Ligandenkomplexe sind, wie dem Fachmann geläufig ist, im Handel erhältlich. Beispiele von Handelsnamen für zinkhaltige Ricionolsäure sind z.B. Tego^{®} Sorb, Tegodeo^{®} der Fa. Goldschmidt AG, DE.

Vorzugsweise enthält die erfindungsgemäße Zusammensetzung die Verbindungen der Formel I bzw. II in einem Gesamtanteil von 1 bis 99 Gew.%.

Der Rest der erfindungsgemäßen Zusammensetzung besteht vorzugsweise, im Falle der Anwesenheit einer Verbindung der Formel I zu 90 Gew.%, insbesondere 95 Gew.%, weiter bevorzugt mindestens 98 Gew.%, insbesondere mindestens 99 Gew.% aus mindestens einem Lösungsvermittler wie hierin beschrieben. Grundsätzlich können auch weitere Hilfs- und Zusatzstoffe eingesetzt werden, die dem Fachmann auf dem jeweiligen Einsatzgebiet bekannt sind und überwiegend CTFA gelistet sind und in den üblichen Mengen eingesetzt werden können.

### Methoden:

### Oberfläche/Porenvolumen:

Die spezifische Oberfläche (BET-Oberfläche) wurde an einem vollautomatischen Stickstoffporosimeter der Firma Micromeritics, Typ ASAP 2010, gemäß DIN 66131 durchgeführt. Das Porenvolumen wurde unter Anwendung der BJH Methode ermittelt (E.P Barrett, L.G. Joyner, P.P. Haienda, J. Am. Chem. Soc. 73 (1951) 373). Porenvolumina bestimmter Porengrößenbereiche werden durch Aufsummieren inkrementeller Porenvolumina bestimmt, die aus der Auswertung der Adsorptionsisotherme nach BJH erhalten werden. Das Gesamtporenvolumen nach der BJH-Methode bezieht sich auf Poren mit einem Durchmesser von 2 bis 130 nm.

### Silicatanalyse:

Diese Analyse beruht auf dem Totalaufschluss des Rohtons bzw. des entsprechenden Produktes. Nach dem Auflösen der Feststoffe werden die Einzelkomponenten mit herkömmlichen spezifischen Analysenmethoden, wie z.B. ICP, analysiert und quantifiziert.

### Ionenaustauschkapazität:

Zur Bestimmung der Ionenaustauschkapazität (IUF) wurde der zu untersuchende Rohton über einen Zeitraum von zwei Stunden bei 105°C getrocknet. Danach wurde das getrocknete Material mit einem Überschuss an wässriger 2N NH₄Cl-Lösung eine Stunde unter Rückfluss zur Reaktion gebracht. Nach einer Standzeit von 16 Stunden bei Raumtemperatur wurde filtriert, worauf der Filterkuchen gewaschen, getrocknet und vermahlen wurde und der NH₄-Gehalt im Rohton durch Stickstoffbestimmung (CHN-Analysator der Fa. Leco) nach den Herstellerangaben ermittelt wurde. Der Anteil und die Art der ausgetauschten Metallionen wurde im Filtrat durch ICP-Spektroskopie bestimmt.

### Bestimmung des Sedimentvolumens

Ein graduierter 100 ml Messzylinder wird mit 100 ml destilliertem Wasser bzw. einer wässrigen Lösung aus 1 % Soda und 2 % Trinatriumpolyphosphat gefüllt. 2 g der zu vermessenden Substanz werden langsam und portionsweise, je etwa 0,1, bis 0,2 g, mit einem Spatel auf die Oberfläche des Wassers gegeben. Nach dem Absinken einer zugegebenen Portion wird die nächste Portion zugegeben. Nachdem die 2 g Substanz zugegeben und auf den Grund des Messzylinders abgesunken sind, wird der Zylinder für eine Stunde bei Raumtemperatur stehen gelassen. Anschließend wird an der Graduierung des Messzylinders die Höhe des Sedimentvolumens in ml/2g abgelesen.

### Bestimmung der Volumenzunahme des Sedimentvolumens (Quellvermögen)

Der wie oben beschrieben zur Bestimmung des Sedimentvolumens verwendete Probenansatz wird mit Parafilm^{®} verschlossen und für drei Tage bei Raumtemperatur erschütterungsfrei stehen gelassen. Danach wird an der Graduierung des Messzylinders das Sedimentvolumen abgelesen. Die Zunahme des Sedimentvolumens ergibt sich aus der Differenz des Sedimentvolumens zu Beginn der Messung und nach einer Standzeit von drei Tagen.

### Röntgendiffraktometrie:

Die Röntgenaufnahmen werden an einem hochauflösenden Pulverdiffraktometer der Fa. Phillips (X'-Pert-MPD(PW 3040)) erstellt, das mit einer Cu-Anode ausgerüstet war.

Die Erfindung wird nun anhand der nachstehenden nicht beschränkenden Beispiele näher erläutert:

Dabei zeigen die nachfolgenden Figuren:
- Fig. 1:: Adsorption von Schwefelwasserstoff, 1 g Adsorbens, 1 ml 1:100 verdünnt gesättigte Natriumsulfidlösung, 100 µl 99% Essigsäure,
- Fig. 2:: Adsorption von Ammoniak, 20 µl 1:2 verdünnt 30% Ammoniaklösung,
- Fig. 3:: Adsorption von Schwefelwasserstoff, 1 g Adsorbens, 1 ml 1:100 verdünnt gesättigte Natriumsulfidlösung, 100 µl 99% Essigsäure,
- Fig. 4:: Adsorption von Ammoniak, 1 g Adsorbens, 10 µl 1:5 verdünnt 30% Ammoniaklösung,
- Fig. 5:: Adsorption von Ammoniak, 50 µl 1:2 verdünnt 30% Ammoniaklösung, 1 g Aktivstoff,
- Fig. 6:: Vergleich der Adsorption von Schwefelwasserstoff, 1 ml Adsorbens, 1 ml 1:100 verdünnt gesättigte Natriumsulfidlösung, 100 µl Essigsäure (99%),
- Fig. 7:: Vergleich der Adsorption von Ammoniak, 1 ml Adsorbens, 10 µl 1:2 verdünnt 30% Ammoniaklösung,
- Fig. 8:: Ammoniakadsorption, 1 ml Adsorbens, 10 µl 1:2 verdünnt 30% Ammoniaklösung,
- Fig. 9:: Schwefelwasserstoffadsorption, 1 ml Adsorbens, 1 ml 1:100 verdünnt gesättigte Natriumsulfidlösung, 100 µl 99% Essigsäure,
- Fig. 10:: Adsorption von Schwefelwasserstoff an Zinksalzen, 1 ml 1:200 verdünnt gesättigte Natriumsulfidlösung, 50 µl 99% Essigsäure,
- Fig. 11:: Adsorption von Ammoniak 10 µl 1:10 äquivalente Zinkmengen,
- Fig. 12:: Vergleich der Adsorption von Schwefelwasserstoff, 5 ml Adsorbens (getrocknet bei 95 °C), 1 ml 1:100 verdünnt gesättigte Natriumsulfidlösung, 100 µl (99%),
- Fig. 13:: Adsorption von Ammoniak, 10 µl 1:10 verdünnt 30% Ammoniaklösung, 1 g Aktivstoff,
- Fig. 14:: Adsorption von Ammoniak, 10 µl 1:5 verdünnt 30% Ammoniaklösung, 1 g Adsorbens,
- Fig. 15:: Adsorption von Schwefelwasserstoff, 1 g Adsorbens, 1 ml 1:100 verdünnt gesättigte Natriumsulfidlösung, 100 µl Essigsäure (99%),
- Fig. 16:: Adsorption von Ammoniak aus Lösung, 1 g Adsorbens, unter Rühren,
- Fig. 17:: Vergleich der Adsorption von Schwefelwasserstoff, 5 ml Adsorbens (getrocknet bei 95 °C), 1 ml 1:100 verdünnt gesättigte Natriumsulfidlösung, 100 µl (99%),

- Fig. 18:: Vergleich der Adsorption von Schwefelwasserstoff, 1 ml Adsorbens, 1 ml 1:100 verdünnt gesättigte Natriumsulfidlösung, 100 µl Essigsäure (99%),
- Fig. 19:: Vergleich der Adsorption von Schwefelwasserstoff, 5 ml Adsorbens, 1 ml 1:100 verdünnt gesättigte Natriumsulfidlösung, 100 µl (99%).
- Fig. 20: a: Adsorption von Schwefelwasserstoff, 1ml 1:100 verdünnte gesättigte Natriumsufidlösung, 100µl 99% Essigsäure; 1ml Adsorbens.

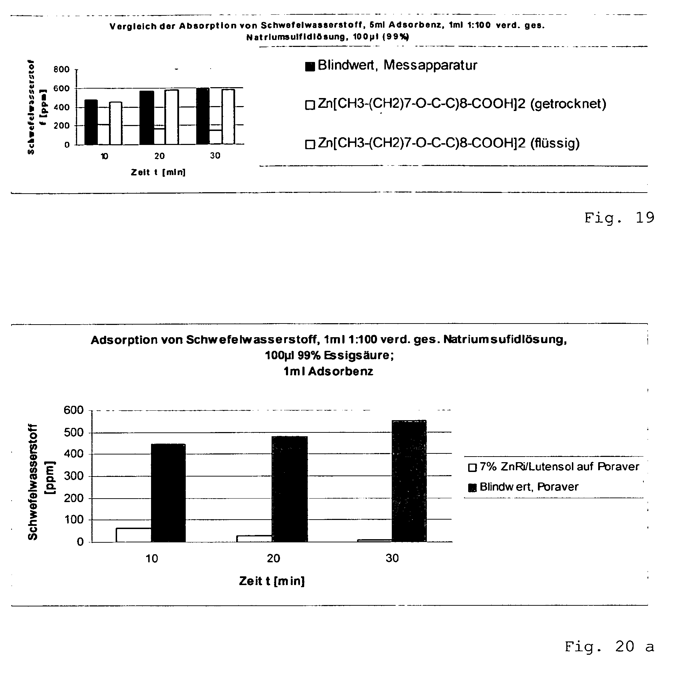

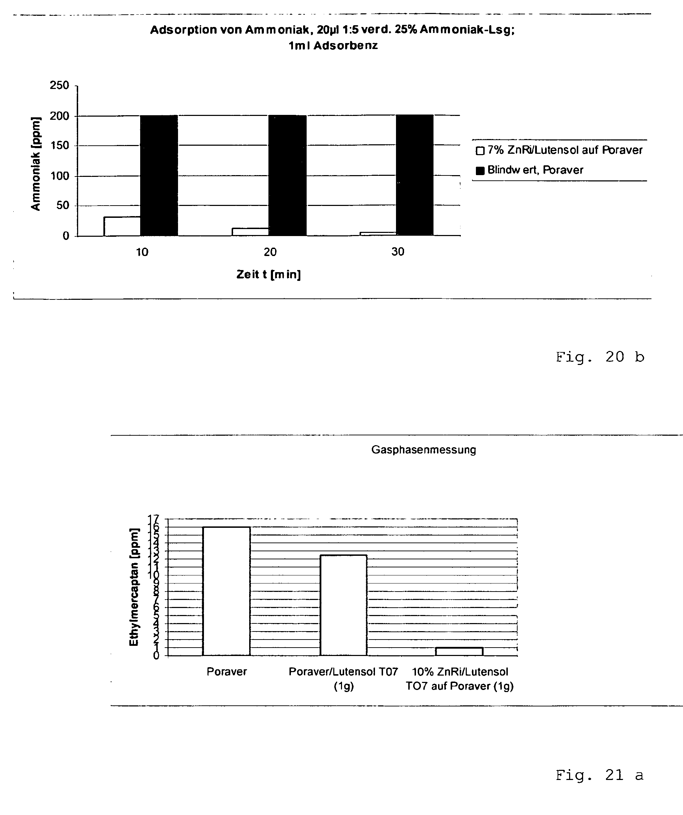

- Fig. 20: b: Adsorption von Ammoniak, 20µl 1:5 verdünnte 25% Ammoniak-Lösung; 1ml Adsorbens

### Versuchsbeschreibung zur Schwefelwasserstoff- und Ammoniak Adsorption:

Verwendete Labor- und Messgeräte für die Schwefelwasserstoff- und Ammoniak-Adsorptionsmessung:
- 500 ml Erlenmeyerkolben (NS 45/40)
- Waschflaschenaufsatz (NS 29/32)
- Reduzierstück H 29/32 - K 45/40
- Rückschlagventil; PE-HD Ventilplättchen aus FKM, z.B. Art.-Nr. GY/09303039 bei Th.Geyer
- 3-Wege-Ventil; z.B. Art.-Nr.: GY/091 16776 bei Th.Geyer
- Petrischale, Durchmesser 3 cm, Höhe 1 cm
- Dräger Messgerät X-am 7000 mit Pumpe und Pumpenadapter
- DrägerSensor XS EC NH₃; Art-Nr. 6809145
- Dräger Sensor XS EC H2S HC; Art.-Nr. 6809180
- Viton-Schlauch für das Dräger Messgerät; Art.-Nr. 1203150
- Naturkautschukschlauch

### Schwefelwasserstoff:

Ein Gramm des zu untersuchenden Substrates wird in einem abgeschlossenen 500ml Erlenmeyerkolben mit Schliff (NS 45/40) und Waschflaschenaufsatz (NS 29/32) vorgelegt. Der obere Kuppel-Ausgang wird über ein zwei Zentimeter langen Naturkautschuk-Schlauch an ein Rückschlagventil angeschlossen. Der in den Erlenmeyerkolben eintauchende Ausgang wird über ein zwei Zentimeter langen Naturkautschuk-Schlauch über ein drei-Wege-Ventil an das Messgerät X-am 7000 angeschlossen.

Eine Petrischale innerhalb des Erlenmeyerkolbens wird mit I ml einer 1:100 bzw. 1:200 mit destilliertem Wasser verdünnten vormals gesättigten Natriumsulfidlösung beschickt und die Lösung mit 100 µl bzw. 50 µl konzentrierter Essigsäure angesäuert und damit die Schwefelwasserstoffentwicklung initiiert.

Die gebildete Schwefelwasserstoff/Luft Atmosphäre wird dann mit Hilfe eines Dräger Schadstoffmessgerätes X-am 7000 und zugehöriger Pumpe komplett über das drei-Wege-Ventil aus dem Erlenmeyerkolben entnommen und der Schwefelwasserstoffanteil der Atmosphäre im ppm-Bereich automatisch durch den elektrochemischen Dräger-Sensor angegeben.

Durch die zeitlich versetzte Probennahme jeweils neuer Proben kann die Dynamik der Schwefelwasserstoffadsorption stoffspezifisch ermittelt werden.

### Ammoniak:

Ein Gramm des zu untersuchenden Substrates wird in einem abgeschlossenen 500 ml Erlenmeyerkolben mit Schliff (NS 45/40) und Waschflaschenaufsatz (NS 29/32) vorgelegt. Der obere Kuppel-Ausgang wird über ein zwei Zentimeter langen Naturkautschuk-Schlauch an ein Rückschlagventil angeschlossen. Der in den Erlenmeyerkolben eintauchende Ausgang wird über ein zwei Zentimeter langen Naturkautschuk-Schlauch über ein drei-Wege-Ventil an das Messgerät X-am 7000 angeschlossen.

Eine Petrischale innerhalb des Erlenmeyerkolbens wird mit einer auf die Azidität der Oberfläche des Substrats abgestimmten, mit destilliertem Wasser verdünnten 30% Ammoniak-Lösung beschickt. Bei stark sauren Oberflächen sollten 50 µl einer 1:2 Verdünnung, bei neutralen Oberflächen 10 µl einer 1:10-Verdünnung der 30% Ammoniak-Lösung verwendet werden. Die jeweils genutzte Verdünnung sollte aber nach Blindwertmessung und nach Ergebnissen/Sensorausschlag des Dräger-Messgerätes zwischen 1:2- und 1:10-Verdünnung gewählt werden.

Um die Verdampfung und somit die Entstehung einer homogenen Gasatmosphäre innerhalb des Glasgefäßes zu ermöglichen, wird die ammoniakalische Lösung auf ein aschefreies Filterpapier in der Petrischale gegeben.

Die gebildete Ammoniak/Luft Atmosphäre wird dann mit Hilfe eines Dräger Schadstoffmessgerätes X-am 7000 und zugehöriger Pumpe komplett über das drei-Wege-Ventil aus dem Erlenmeyerkolben entnommen und der Ammoniakanteil der Atmosphäre im ppm-Bereich automatisch durch den elektrochemischen Dräger-Sensor angegeben.
Durch die zeitlich versetzte Probennahme jeweils neuer Proben kann die Dynamik der Ammoniakadsorption stoffspezifisch ermittelt werden.

Es wurden folgende Materialien als feste Träger für die erfindungsgemäßen Formulierungen verwendet:
- KIESELGEL 60, 0,063-0,200 mm: Carl Roth GmbH + Co. KG, Karlsruhe, DE
- Aluminiumoxid-90-Neutral: Carl Roth GmbH + Co. KG, Karlsruhe, DE
- Titandioxid P805: Sigma-Aldrich Chemie GmbH, Taufkirchen, DE
- Laundrosil® 214: Süd-Chemie AG, Moosburg, DE
- Poraver-Glaskugeln (0.1-0.3mm): Dennert Poraver GmbH, Schlüsselfeld, DE

Im Folgenden wird die Abkürzung ZnRi für Zinkricinoleat verwendet. Zinkricinoleat in der wässrigen Formulierung TegoSorb A30 ist bei Degussa-Goldschmidt (DE) kommerziell erhältlich.
ZnRi kann auch als feste Reinsubstanz von Degussa-Goldschmidt (DE) oder von der Firma Franken-Chemie (DE) bezogen werden unter dem Handelsnamen PY88.

In den nachstehenden Diagrammen ist immer die vorhandene Menge des Schadstoffes in der Atmosphäre gegenüber der Beprobungszeit aufgetragen. Es ist jeweils der verwendete feste Träger, der darauf aufgebrachte Wirkstoff und der getestete Schadstoff angegeben.

### Beispiel 1:

Medium: Fester Träger: Kieselgel. Adsorption aus der Gasphase
- Wirkstoff:: ZnRi*Et-OH
- Schadstoff:: H₂S

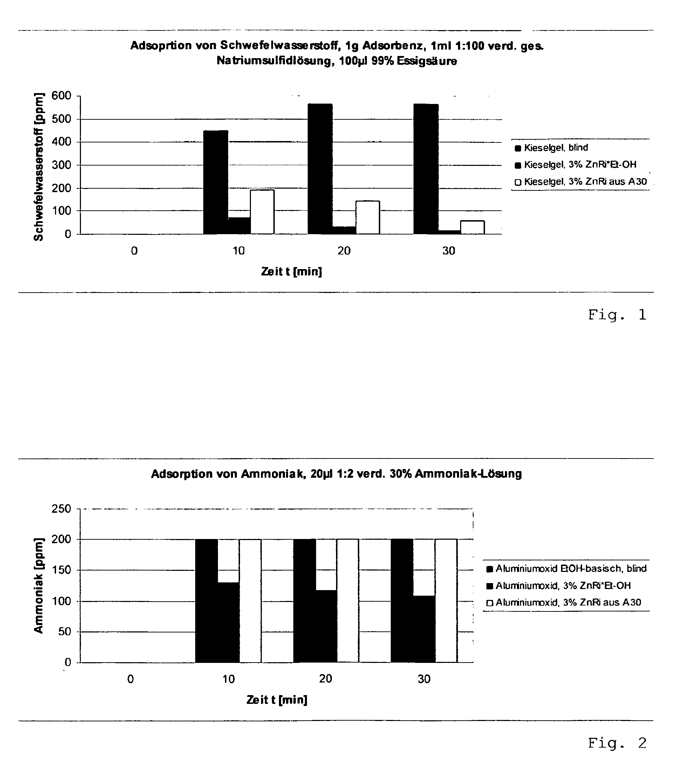

Die Ergebnisse sind in Fig. 1 dargestellt.

Wie daraus ersichtlich ist, zeigt das erfindungsgemäße ZnRi*Et-OH mit Abstand die beste Aktivität. Tegosorb A30 zeigt eine deutlich geringere Aktivität.

### Beispiel 2:

Medium: Fester Träger: Aluminiumoxid. Adsorption aus der Gasphase
- Wirkstoff:: ZnRi*Et-OH
- Schadstoff:: NH₃

Die Ergebnisse sind in Fig. 2 dargestellt.

Wie daraus ersichtlich ist, zeigt das erfindungsgemäße ZnRi*Et-OH die beste Aktivität. Tegosorb A30 zeigt keine Aktivität.

### Beispiel 3:

Medium: Fester Träger: Aluminiumoxid. Adsorption aus der Gasphase
- Wirkstoffe:: ZnRi*Et-OH
- Schadstoff:: H₂S

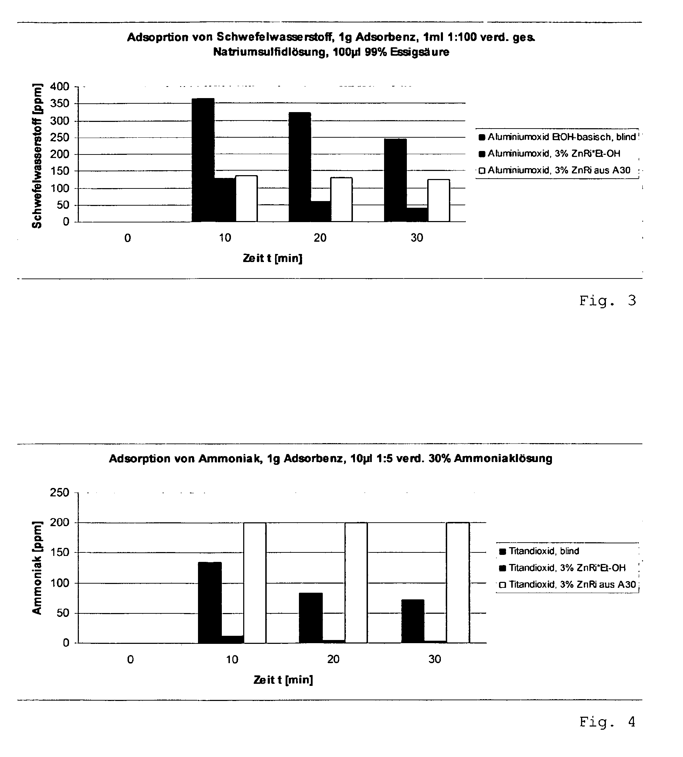

Die Ergebnisse sind in Fig. 3 dargestellt.

Wie daraus ersichtlich ist, zeigt das erfindungsgemäße ZnRi*Et-OH die beste Aktivität. Tegosorb A30 zeigt eine deutlich geringere Aktivität.

### Beispiel 4:

Medium: Fester Träger: Titandioxid. Adsorption aus der Gasphase
- Wirkstoffe:: ZnRi*Et-OH
- Schadstoff:: NH₃

Die Ergebnisse sind in Fig. 4 dargestellt.

Wie daraus ersichtlich ist, zeigt das erfindungsgemäße ZnRi*Et-OH die beste Aktivität. Tegosorb A30 zeigt eine deutlich geringere Aktivität.

### Beispiel 5:

Medium: Fester Träger: Schichtsilikat Laundrosil 214. Adsorption aus der Gasphase
- Wirkstoffe:: ZnRi₂*Et-OH
- Schadstoff:: NH₃

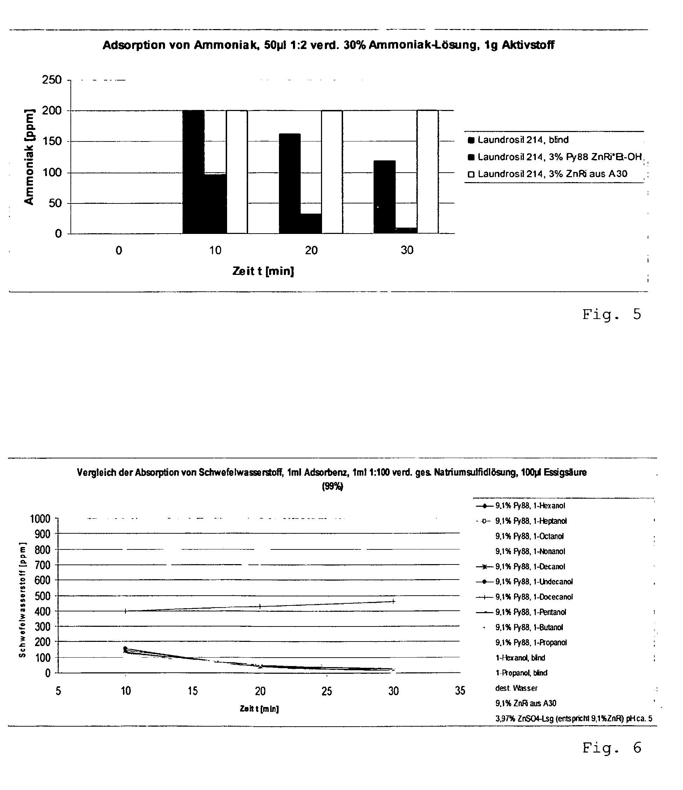

Die Ergebnisse sind in Fig. 5 dargestellt.

Wie daraus ersichtlich ist, zeigt das erfindungsgemäße ZnRi*Et-OH die beste Aktivität. Tegosorb A30 zeigt eine deutlich geringere Aktivität.

### Beispiel 6:

Medium: Alkoholische Lösungen. Adsorption aus der Gasphase
- Wirkstoffe:: ZnRi*R-OH (R=C₃-C₁₂) (PY88 = ZnRi)
- Schadstoff:: H₂S

Die Ergebnisse sind in Fig. 6 dargestellt.

Wie daraus ersichtlich ist sind die erfindungsgemäßen Zusammensetzungen ZnRi*R-OH (R=C₃-C₁₂) eine gute Aktivität gegenüber H₂S. ZnSO₄-Lösung als Referenzsystem sowie die Alkohole als Blindwert sind inaktiv.

### Beispiel 7:

Medium: Alkoholische Lösungen. Adsorption aus der Gasphase
- Wirkstoffe:: ZnRi*R-OH (R=C₃-C₁₂) (PY88 = ZnRi)
- Schadstoff:: NH₃

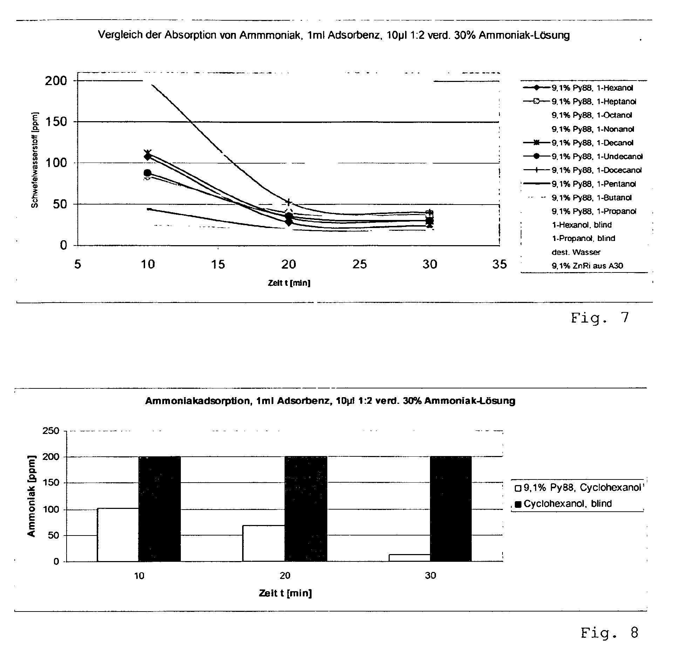

Die Ergebnisse sind in Fig. 7 dargestellt.

Wie daraus ersichtlich ist sind die erfindungsgemäßen Zusammensetzungen ZnRi*R-OH (R=C₃-C₁₂) eine gute Aktivität gegenüber NH₃. Die Alkohole als Blindwert sind inaktiv. Tegosorb A30 zeigt kaum Aktivität.

### Beispiel 8:

Medium: Cyclohexanol Lösung. Adsorption aus der Gasphase
- Wirkstoff:: ZnRi *C₆H₁₂-OH (PY88 = ZnRi)
- Schadstoff:: NH₃

Die Ergebnisse sind in Fig. 8 dargestellt.

Schlussfolgerung: Das erfindungsgemäße ZnRi*C₆H₁₂-OH ist aktiv gegenüber NH₃.

### Beispiel 9:

Medium: Cyclohexanol Lösung. Adsorption aus der Gasphase
- Wirkstoff:: ZnRi *C₆H₁₂-OH (PY88 = ZnRi)
- Schadstoff:: H₂S

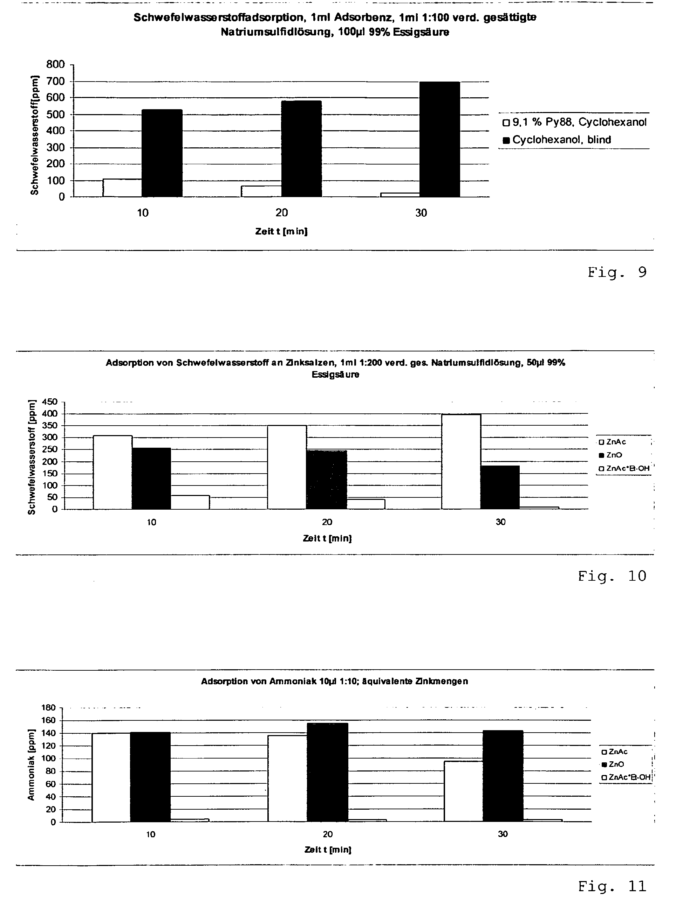

Die Ergebnisse sind in Fig. 9 dargestellt.

Schlussfolgerung: Das erfindungsgemäße ZnRi*C₆H₁₂-OH ist aktiv gegenüber H₂S.

### Beispiel 10:

Medium: Feststoffe. Adsorption aus der Gasphase
- Wirkstoffe:: ZnAc*Et-OH (Ac = CH3-COO⁻)
- Schadstoff:: H₂S

Die Ergebnisse sind in Fig. 10 dargestellt.

Schlussfolgerung: Das erfindungsgemäße ZnAc*Et-OH ist aktiv gegenüber H₂S. Die Aktivität ist deutlich besser als bei dem Referenzsystem ZnO. Reines ZnAc zeigt keine Aktivität.

### Beispiel 11:

Medium: Feststoffe. Adsorption aus der Gasphase
- Wirkstoff:: ZnAc *Et-OH (Ac = CH3-COO⁻)
- Schadstoff:: NH₃

Die Ergebnisse sind in Fig. 11 dargestellt.

Schlussfolgerung: Das erfindungsgemäße ZnAc*Et-OH ist hoch aktiv gegenüber NH₃. Die Aktivität ist deutlich besser als bei ZnAc, welches eine geringe Aktivität aufweist. ZnO zeigt keine Aktivität.

### Beispiel 12:

Medium: Reine Feststoffe. Adsorption aus der Gasphase
- Wirkstoffe:: ZnRi *Et-OH
- Schadstoff:: H₂S

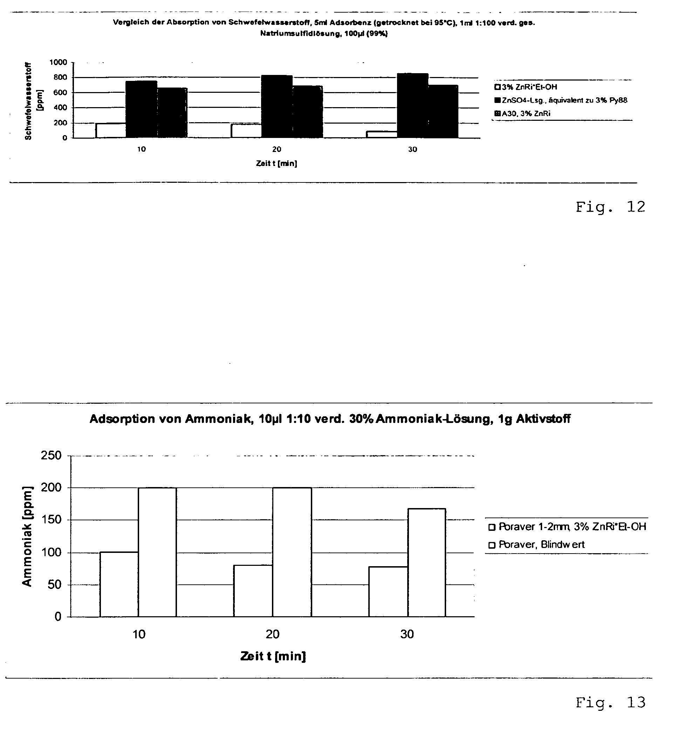

Die Ergebnisse sind in Fig. 12 dargestellt.

Schlussfolgerung: Das erfindungsgemäße ZnRi*Et-OH wurde hier als reinen Feststoff durch Abdampfen von Ethanol hergestellt. Die Erfindung zeigt deutliche Aktivität. Die eingedampfte wässrige Vergleichs-Formulierung Tegosorb A30 (ZnRi aus A30) sowie ZnSO₄ zeigen keine Aktivität.

### Beispiel 13:

Medium: Wirkstoff auf festem Trägermaterial. Adsorption aus der Gasphase
- Adsorbens:: Poraver-Glas
- Wirkstoffe:: ZnRi *Et-OH
- Schadstoff:: NH₃

Die Ergebnisse sind in Fig. 13 dargestellt.

Die erfindungsgemäße Zusammensetzung zeigt deutliche Aktivität. Reines Poraver (Träger) ist nur sehr geringfügig aktiv.

### Beispiel 14:

Medium: Wirkstoff auf festem Trägermaterial. Adsorption aus der Gasphase
- Adsorbens:: Poraver-Glas
- Wirkstoffe:: ZnAc*Et-OH, ZnAc*Me-OH
- Schadstoff:: NH₃

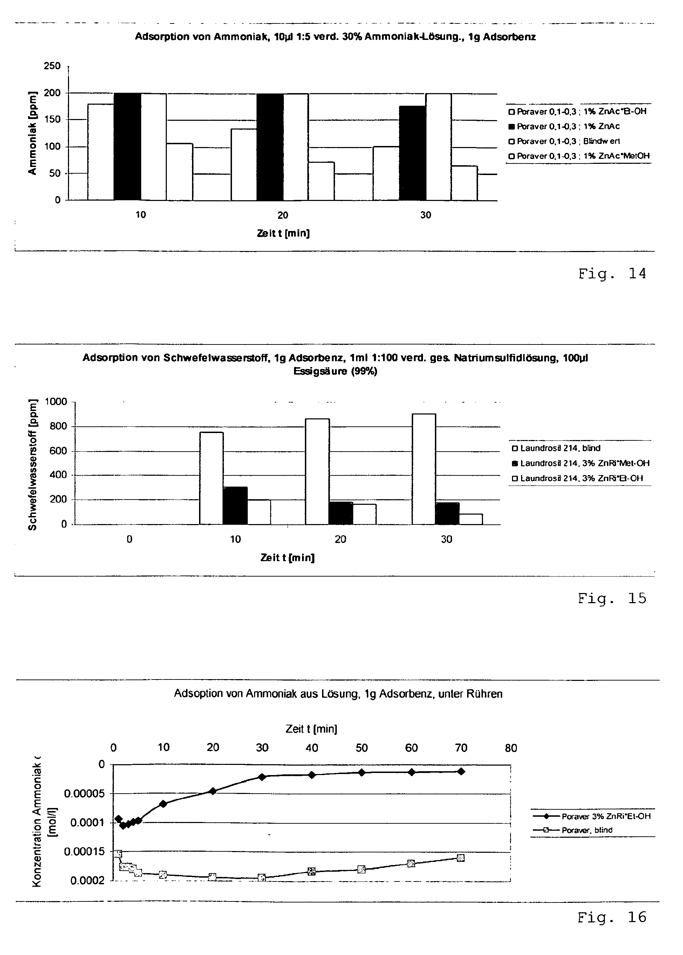

Die Ergebnisse sind in Fig. 14 dargestellt.

Die erfindungsgemäße Zusammensetzung zeigt deutliche Aktivität. Reines Poraver (Träger) und reines ZnAc sind nahezu inaktiv.

### Beispiel 15:

Medium: Wirkstoffe auf festem Trägermaterial. Adsorption aus der Gasphase
- Adsorbens:: Laundrosil 214
- Wirkstoffe:: ZnRi *Me-OH und ZnRi₂ *Et-OH
- Schadstoff:: H₂S

Die Ergebnisse sind in Fig. 15 dargestellt.

Schlussfolgerung: Das mit den erfindungsgemäßen Zusammensetzungen ZnRi*Me-OH und ZnRi*Et-OH belegte Tonmineral zeigt deutliche Aktivität. Das Trägermaterial ist als Reinstoff inaktiv gegenüber H₂S. Erst die Belegung aktiviert das Tonmineral.

### Beispiel 16:

Medium: Wirkstoffe auf festem Trägermaterial. Adsorption aus wässriger Lösung
- Adsorbens:: Poraver-Glas
- Wirkstoff:: ZnRi *Et-OH
- Schadstoff:: NH₃

Die Ergebnisse sind in Fig. 16 dargestellt.

Aufgetragen ist die Konzentration von Ammoniak in wässriger Lösung in Abhängigkeit der Zeit bei zugesetztem mit Wirkstoff aktiviertem Adsorbens.
Porver,blind: NH₃-Lösung mit nicht aktivertem Poraver Schlussfolgerung: Das mit der Erfindung ZnRi*Et-OH belegte Porver-Glasmaterial absorbiert NH₃ aus wässriger Lösung. Das Trägermaterial ist als Reinstoff nahezu inaktiv. #

### Beispiel 17:

- Medium:: Wirkstoffe getrocknet
- Wirkstoff:: ZnRi*ET-OH, ZnRi*CH₃-(CH₂)₇-O-C-C)₈-COOH
- Schadstoff:: H₂S

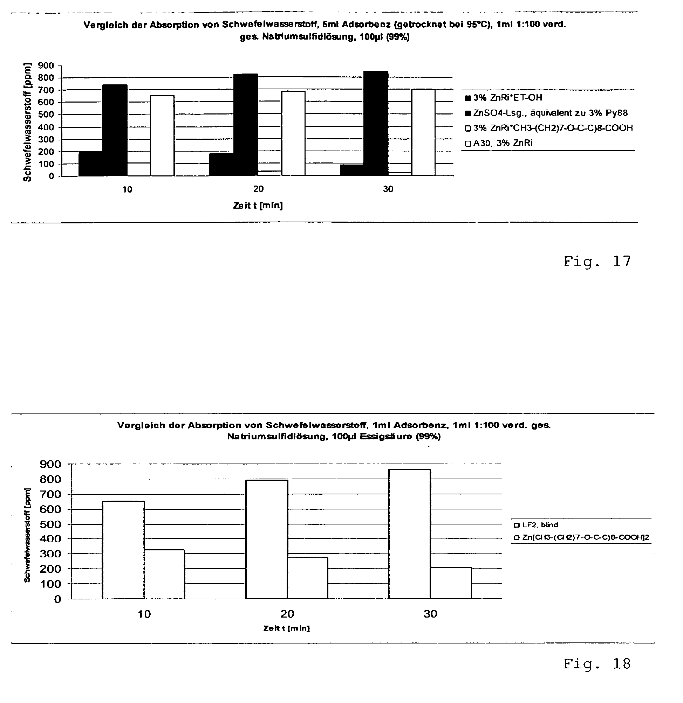

Die Ergebnisse sind in Fig. 17 dargestellt.

Schlussfolgerung: Die erfindungsgemäßen Zusammensetzungen ZnRi*CH₃-(CH₂)₇-O-C-C)₈-COOH und ZnRi*ET-OH sind in getrocknetem Zustand aktiv. Die beste Aktivität zeigt ZnRi*CH₃-(CH₂)₇-O-C-C)₈-COOH. Das kommerzielle Produkt A30 und ZnSO₄ sind im getrockneten Zustand als Feststoffe nicht aktiv.

### Beispiel 18:

- Medium:: Wirkstoff als wasserfreies Konzentrat
- Wirkstoff:: Zn[CH₃-(CH₂)₇-O-C-C)₈-COOH]₂
- Schadstoff:: H₂S

Referenzsystem: Der Ligand zum Vergleich als Konzentrat

Die Ergebnisse sind in Fig. 18 dargestellt.

Schlussfolgerung: Die erfindungsgemäße Zusammensetzung zeigt Aktivität. Der Ligand als reine Ethercarbonsäure ist inaktiv.

### Beispiel 19:

Medium: Wirkstoffe auf Poraver-Material. Adsorption aus der Gasphase
- Wirkstoff:: Zn[CH₃-(CH₂)₇-O-C-C)₈-COOH]₂
- Schadstoff:: H₂S
- Referenzsystem:: Zn[CH₃-(CH₂)₇-O-C-C)₈-COOH]₂ in wässriger Lösung

Die Ergebnisse sind in Fig. 19 dargestellt.

Schlussfolgerung: Die erfindungsgemäße Zusammensetzung Zn[CH₃-(CH₂)₇-O-C-C)₈-COOH]₂ ist nur getrocknet aktiv, nicht in wässriger Lösung.

### Beispiel 20:

In-Situ Herstellung der wasserfreien Wirkstoffe verbunden mit dem Aufbringen auf festen Trägermaterialien

Die Vorgehensweise bei diesem Herstellungsverfahren wurde bereits in der vorstehenden Beschreibung erläutert.
Beispielhaft wird im folgenden allgemein die Herstellung des Addukt-Wirkstoffes (Ri)₂Zn-Lutensol® TO7 aus Rizinolsäure, Zinksulfat und dem Fettalkohol Lutensol^{®}TO7 (Alkohol Ethoxylat 7EO, BASF AG, Ludwigshafen, DE) sowie die Beschichtung eines Trägers (7 Gew.-% Belegung) beschrieben:

1,5g Rizinolsäure (0,005mol) werden mit ca. 15ml Wasser gerührt und auf 75°C erhitzt. Anschließend wird 6M Natronlauge so lange tropfenweise zugegeben bis eine klare Lösung resultiert (pH ca. 8,1). Dann werden 15ml Lutensol T07 unter Rühren beigefügt. Sofort werden 0,725g (0,0025mol) Zinksulfat Heptahydrat, gelöst in 10ml Wasser, mit Hilfe einer Pipette tropfenweise zugeführt.
In die entstandene Suspension werden dann 20g Poraver (0.1-0.3mm) zugemischt und das Wasser anschließend langsam bei 80 °C verdampft. Die Belegung des Festkörpermaterials entspricht einem Wirkstoffgehalt von 7 Gew. %.

Das hergestellte Material wurde mit den vorstehend beschriebenen Methoden und Verfahren auf die Wirksamkeit hinsichtlich der Absorption von NH₃ und H₂S untersucht.

Die Ergebnisse sind in Fig. 20 a und b dargestellt.

Schlussfolgerung: Die Ergebnisse zeigen, dass mit dem hier beschriebenen Verfahren ein wasserfreies aktives Wirkstoff-Addukt auf dem Trägermaterial hergestellt wurde.

### Beispiel 21:

Adsorption von Ethylmercaptan (C₂H₅-SH) mit wasserfreien Wirkstoffaddukten auf festen Trägermaterialien: Adsorption aus der Gasphase und aus unpolaren organischen Lösungsmitteln (iso-Octan)

### a) Versuchsvorschrift zur Adsorption von Ethylmercaptan aus der Gasphase

Ein Gramm mit ZnRi-Lutensol-Addukt belegtes Poraver (0.1-0.3mm) werden in einem abgeschlossenen 500ml Erlenmeyerkolben mit Schliff und Waschflaschenaufsatz vorgelegt. Die beiden Ausgänge des Waschflaschenaufsatzes werden über einen gegen Lösungsmittel inerten Schlauch verbunden, damit aus der Messapparatur kein Gas nach außen dringen kann. Ein weiteres Gefäß innerhalb des Glasbehälters wird mit 100µl einer Ethylmercaptan-Lösung (450 µl Ethylmercaptan in 50g iso-Octan) beschickt. Um die Verdampfung und somit die Entstehung einer homogenen Gasatmosphäre innerhalb des Glasgefäßes zu ermöglichen, wird die Ethylmercaptan- Lösung auf ein aschefreies Filterpapier in einer Petrischale innerhalb des Erlenmeyerkolbens gegeben. Nach zehn Minuten wird die gebildete Ethylmercaptan/Luft-Atmosphäre mit Hilfe eines Dräger Schadstoffmessgerätes X-am 7000 (Drägerwerk AG, Lübeck, DE) und zugehöriger Pumpe komplett aus dem Glasgefäß entnommen und der Ethylmercaptananteil der Atmosphäre im ppm-Bereich durch einen elektrochemischen Drägersensor ((Drägerwerk AG, Lübeck, DE; Artikelnummer 6809200) gemessen.

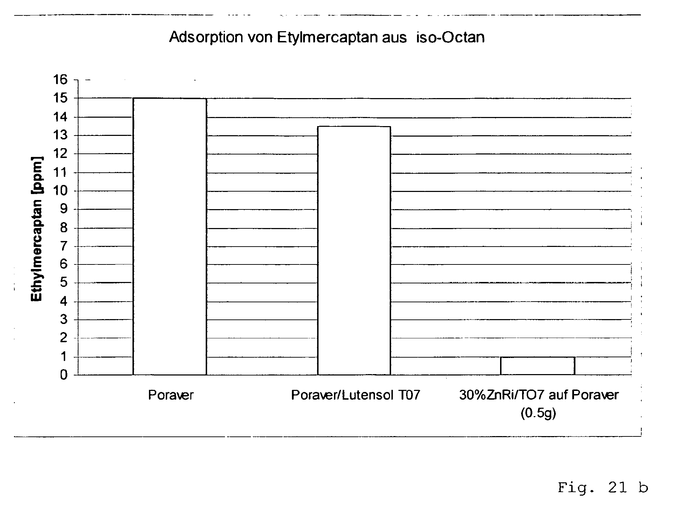

Die Ergebnisse sind in Fig. 21 a dargestellt.

Schlussfolgerung: Das wasserfreie Wirkstoff-Addukt ZnRi/Lutensol auf dem Träger bindet effektiv Ethylmercaptan aus der Gasatmosphäre.

### b) Versuchsvorschrift zur Adsorption von Ethylmercaptan aus einem Lösungsmittel, hier iso-Octan

0.5 Gramm mit ZnRi-Lutensol-Addukt belegtes Poraver werden innerhalb eines abgeschlossenen 500ml Erlenmeyerkolben mit Schliff und Waschflaschenaufsatz in einem weiteren Gefäß innerhalb des Erlenmeyerkolbens vorgelegt. Die beiden Ausgänge des Waschflaschenaufsatzes werden über einen gegen Lösungsmittel inerten Schlauch verbunden, damit aus der Messapparatur kein Gas nach außen dringen kann. 100µl einer Ethylmercaptan-Lösung (450 µl Ethylmercaptan in 50g iso-Octan) werden dann sofort direkt auf die Schüttung im Gefäß gegeben. Nach zehn Minuten wird die gebildete Ethylmercaptan/Luft Atmosphäre mit Hilfe eines Dräger Schadstoffmessgerätes X-am 7000 ((Drägerwerk AG, Lübeck, DE) und zugehöriger Pumpe komplett aus dem Glasgefäß entnommen und der Ethylmercaptananteil der Atmosphäre im ppm-Bereich automatisch durch ein Dräger-Messgerät X-am 7000 und dem elektrochemischen Drägersensor (Drägerwerk AG, Lübeck, DE; Artikelnummer 6809200) gemessen.

Die Ergebnisse sind in Fig. 21b dargestellt.

Schlussfolgerung: Das wasserfreie Wirkstoff-Addukt ZnRi/Lutensol auf dem Träger bindet effektiv Ethylmercaptan aus iso-Octan.

## Patentansprüche

1. Zusammensetzung zur Adsorption von Schadstoffen und/oder Geruchsstoffen, wobei die Zusammensetzung im wesentlichen wasserfrei ist und die folgenden Komponenten enthält:
a1) mindestens ein Metallion in Form eines Ligandenkomplexes, und
a2) mindestens einen Lösungsvermittler, in dem der Ligandenkomplex des Metallions vollständig löslich ist;
oder
b) ein Zinkethercarboxylat.

2. Zusammensetzung nach Anspruch 1, **dadurch gekennzeichnet, dass** der Anteil an Wasser bezogen auf die Gesamtzusammensetzung bei weniger als 5 Gew.-%, insbesondere weniger als 4 Gew.-%, insbesondere weniger als 3 Gew.-%, insbesondere weniger als 2 Gew.-%, insbesondere weniger als 1 Gew.-%, insbesondere weniger als 0,5 Gew.-%, insbesondere weniger als 0,2 Gew.-%, insbesondere weniger als 0,1 Gew.-% liegt.

3. Zusammensetzung nach einem der vorhergehenden Ansprüche, **dadurch gekennzeichnet, dass** das Metallion Zink, Kupfer, Eisen, Cadmium, Quecksilber, Molybdän, Blei, Cobalt, Nickel, Chrom, Vanadium und Wolfram, und insbesondere Zink darstellt.

4. Zusammensetzung nach einem der vorhergehenden Ansprüche, **dadurch gekennzeichnet, dass** der Ligand eine hydroxylierte, ungesättigte und/oder gesättigte Fettsäure oder Acetat, bevorzugt Ricinoleat darstellt, oder allgemein funktionalisierte Fettsäuren, die folgende funktionelle Gruppen enthalten können: -OH, -SO₃⁻, SO₃H, -SO₄⁻, SO₄H, -COO⁻, -COOH, -PO₃⁻ PO₃H, PO₄⁻, PO₄H, -O-(C-C-O-)ₙ-C-C-OH (n=1, 2, ....N), -SH, -S⁻, -SR, NO₂, NO₃⁻, -NH₂, -NHR, -NR₂, -NH₃⁺, - NH₂R⁺, -NHR₂⁺, Glykole, Glycerin, Polyglykole, Polyglycerine, F, Cl, I, Br, oder Sulfate, z.B. Ethersulfate, Sulfonate, Phosphate und Phosphonate.

5. Zusammensetzung nach einem der vorhergehenden Ansprüche, **dadurch gekennzeichnet, dass** es sich bei den Schadstoffen und/oder Geruchsstoffen um Ammoniak, Schwefelwasserstoff, Amine, Thiole, Thioether, Carbonsäuren, NOₓ oder ionische Verbindungen wie NO₃⁻, NO₂⁻, PO₄³⁻, H₂PO₄⁻, HPO₄²⁻, Sulfonate, Sulfite und Sulfate oder aliphatische und aromatische Phosphorverbindungen handelt.

6. Zusammensetzung nach einem der vorhergehenden Ansprüche, **dadurch gekennzeichnet, dass** es sich bei dem Lösungsvermittler um einen Alkohol, insbesondere einen primären oder sekundären Alkohol mit 1 bis 12 Kohlenstoffatomen, nichtionische oder ionische Tenside wie Alkoxylate, Polyglycerine, Glykolether, Glykole, Polyethylenglykole, Polypropylenglykole, Polybutylenglykole, Glycerinesterthoxylate, Polysorbate, Alkylethersulfaten, Alkyl- und/oder Arylsulfonaten, Akylsulfaten, Estersulfonate (Sulfofettsäureester), Lingninsulfonate, Fettsäurecyanamide, anionische Sulfobernsteinsäuretenside, Fettsäureisethionate, Acylaminoalkansulfonate (Fettsäuretauride), Fettsäuresarcosinate, Ethercarbonsäuren oder Alkyl(ether)-Phosphate, gegebenenfalls einseitig mit einem C₁-C₆-Alkanol veretherte, C₂-C₆-Alkylenglykole und Poly-C₂-C₃-alkylenglykolether mit durchschnittlich 1 bis 9 gleichen oder verschiedenen, vorzugsweise gleichen Alkylenglykolgruppen pro Molekül, Fettalkoholpolyglykolether, vorzugsweise Propylenglykol, Dipropylenglykol, Trimethylolpropan sowie niederethoxyilierte Fettalkohole mit 6 bis 22, vorzugsweise 8 bis 18, insbesondere 8 bis 12 und besondere bevorzugt 8 bis 11 Kohlenstoffatomen, oder deren Mischungen handelt.

7. Zusammensetzung nach einem der vorhergehenden Ansprüche, **dadurch gekennzeichnet, dass** es sich bei dem Lösungsvermittler um einen primären oder sekundären Alkohol mit 1 bis 4 Kohlenstoffatomen, insbesondere um Ethanol oder Methanol handelt.

8. Zusammensetzung nach einem der vorhergehenden Ansprüche, **dadurch gekennzeichnet, dass** die Zusammensetzung in geträgerter Form vorliegt.

9. Zusammensetzung nach einem der vorhergehenden Ansprüche, **dadurch gekennzeichnet, dass** als Träger Glas (Poraver), natürliche und synthetische Schichtsilicate, Kieselgele, Aluminiumoxide, Titanoxide, Aktivkohle, poröse Kunststoffe oder deren Gemische eingesetzt werden.

10. Zusammensetzung nach einem der vorhergehenden Ansprüche, **dadurch gekennzeichnet, dass** das Schichtsilikat ausgewählt ist aus der Gruppe der Zweischichtsilicate, insbesondere Chyrsotil, Antigorit, Kaolinit, Halloysit und Dreischichtsilicate, insbesondere Talk, Pyrophyllit, Saponit, Hectorit, Montmorillonit, Beidellit, Nontronit, Biotit, Phlogopit, Lepidolith, Muskovit, Paragonit, Margarit und Chlorit, oder deren Mischungen.

11. Zusammensetzung nach einem der vorhergehenden Ansprüche, **dadurch gekennzeichnet, dass** das Schichtsilicat eine Schichtsilicat-Kieselgel-Mischphase darstellt.

12. Verfahren zur Herstellung einer Zusammensetzung zur Adsorption von Schadstoffen und/oder Geruchsstoffen gemäß Anspruch 1 a), umfassend mindestens die folgenden Schritte:
a) Bereitstellen mindestens eines Metallions in Form eines Ligandenkomplexes, und
b) Bereitstellen mindestens eines Lösungsvermittlers, in dem der Ligandenkomplex des Metallions vollständig löslich ist;
c) In Kontaktbringen der Komponenten a) und b).

13. Verfahren nach Anspruch 12, **dadurch gekennzeichnet, dass** die Bereitstellung des Metallions in Form eines Ligandenkomplexes (Komponente a) gemäß Anspruch 12) in wässriger bzw. wasserhaltiger Lösung erfolgt, ein Lösungsvermittler (Komponente b) gemäß Anspruch 12) zugegeben wird, und Wasser anschließend entfernt, insbesondere abgedampft wird, vorzugsweise in Anwesenheit mindestens eines Trägers.

14. Verfahren nach Anspruch 12 oder 13, **dadurch gekennzeichnet, dass** ein Lösungsvermittler eingesetzt wird, der einen höheren Siedepunkt aufweist als Wasser, insbesondere ein hochsiedender Alkohol.

15. Verfahren zur Herstellung einer Zusammensetzung zur Adsorption von Schadstoffen und/oder Geruchsstoffen gemäß Anspruch 1 b), umfassend mindestens die folgenden Schritte:
a) Bereitstellen eines basischen Zinkcarbonats, und
b) Lösen der Komponente a) in mindestens einer Ethercarbonsäure.

16. Verwendung der Zusammensetzung nach einem der Ansprüche 1 bis 11 oder herstellbar nach einem der Ansprüche 12 bis 15 zur Adsorption von Schad- und/oder Geruchsstoffen aus flüssigen oder gasförmigen Medien, insbesondere zur Adsorption von flüchtigen Schwefel-, und/oder Stickstoff- und/oder Carbonsäureverbindungen, insbesondere Ammoniak, Schwefelwasserstoff, Aminen, Thiolen, Thioethern, Carbonsäuren, NOₓ oder auch ionische Verbindungen wie NO₃⁻, NO₂⁻, PO₄³⁻, H₂PO₄⁻, HPO₄²⁻, vorzugsweise zur Adsorption von H₂S und/oder NH₃, und/oder
in Luftfilteranlagen, besonders bevorzugt in Automobilen, und/oder
in Waschmittelzusammensetzungen.
